# EUROPEAN PATENT SPECIFICATION

(11) **EP 0 948 338 B1**
(45) Date of publication and mention of the grant of the patent: **31.07.2002**
(21) Application number: 97913546.4
(22) Date of filing: 18.11.1997
(51) Int. Cl.: A61K 35/54

(54) **UTILIZATION OF AN EGG-BASED PREPARATION AS AN ANTIDEPRESSANT**
Verwendung einer Zusammensetzung auf Eierbasis als Antidepressiva
UTILISATION D'UNE PREPARATION A BASE D'OEUF EN TANT QU'ANTIDEPRESSEUR

(30) Priority: 20.11.1996 NO 964935
(43) Date of publication of application: 13.10.1999
(73) Proprietor: DRYMED A/S, 1254 Oslo (NO)
(72) Inventor: ESKELAND, Bjodne, N-1430 As (NO)
(74) Representative: Bergvall-Eftring, Stina Lena
(86) International application number: NO9700305
(87) International publication number: WO9822119

(56) References cited:
- EP-A- 0 074 251
- EP-A- 0 347 899
- WO-A-94/03192

## Description

The present invention relates to the use of an egg-based preparation as an antidepressant.

More specifically, the invention relates to the utilization of a preparation corresponding to the one described in NO 922988 and the WO 94/03192 based thereon, as an antidepressant.

In NO application 922988 there is described a potency enhancing agent based on a powder produced from fertilized avian eggs.

The application is directed particularly toward a powder of eggs obtained 10 days after fertilization.

In accordance with NO application 962886, the egg preparation corresponding to the ones in NO 922988 also seemed to have a muscle building and strength enhancing effect when used.

Further investigations seem to suggest that preparations of this type also have an antidepressant effect.

Preliminary studies carried out on persons for whom antidepressant medications had not been prescribed were examined over an 8-week period, with results which were clearly indicative of an antidepressant effect.

A study was thus carried out on 6 mentally depressed persons.

These subjects were administered the product in an amount of 3 g x 2 per day for 8 weeks.

The product is based on avian eggs which have been fertilized and incubated for about 10 days.

The three criteria which the subjects were asked to evaluate were:
1) self-esteem,
2) well-being ("happiness"), and
3) energy level.

The individuals were asked to evaluate the three factors of self-esteem, well-being and energy level each week for 8 weeks, utilizing a visual analogue scale ranging from 0 cm = no change, to 10 cm = very pronounced change, said scale having a midpoint of 5 representing the normal condition.

The results are summarized in Table 1.

**Table 1**

| Changes in self-esteem, well-being and energy level before and after an 8-week treatment. | | |
|---|---|---|
| | Before | After 8 weeks |
| Self-esteem | 1.8 +/- 0.3 | 6.4 +/- 0.8 |
| Well-being | 1.1 +/- 0.3 | 5.9 +/- 0.7 |
| Energy level | 2.8 +/- 0.7 | 4.6 +/- 1.0 |

In the course of the 8-week periods, the participants in this study consumed the recommended daily dosage; and, as is indicated in the table, the level of self-esteem increased by a factor of 3.5, well-being increased by a factor of 5.4 and energy level rose by a factor of 1.6.

The weekly change was examined, and these results are given in Table 2.

**Table 2**

| Response on a weekly basis for 8 weeks when 6 individuals were given the product. | | | | | | | |
|---|---|---|---|---|---|---|---|
| | Weeks of treatment | | | | | | |
| | 0 | 1 | 2 | 3 | 4 | 6 | 8 |
| Self-esteem | 1.8 | 1.8 | 2.8 | 3.1 | 4.0 | 5.1 | 5.9 |
| Well-being | 1.1 | 1.6 | 2.6 | 3.9 | 4.1 | 5.0 | 5.9 |
| Energy level | 2.8 | 2.9 | 3.4 | 4.4 | 4.4 | 4.6 | 4.6 |

For the three criteria measured, it is apparent from Table 2 that a gradual increase occurred after the first week and throughout the entire test period of 8 weeks.

The values reported above are the average of what was obtained from the 6 subjects. The conclusion from this, based on the judgments for 1 to 3 above, is that the evaluated product clearly seems to have a significant antidepressant effect.

The product may be consumed as is, but it may also advantageously be ingested with the aid of one or more physiologically acceptable carriers or fillers.

## Claims

1. The use of dried, fertilized chicken-eggs which have been incubated for about 10 days for the production of a preparation for use as an antidepressant.

## Patentansprüche

1. Anwendung von trockenen, befruchteten Hühnereiern, welche ungefähr 10 Tage bebrütet wurden, für die Herstellung einer Zubereitung für Anwendung als Antidepressivum.

## Revendications

1. L'utilisation d'oeufs de poulet fertilisés et sechés ayant été incubés pendant 10 jours, pour la production d'une préparation pour l'utilisation comme un antidépresseur.
